# EUROPEAN PATENT SPECIFICATION

(11) **EP 2 299 923 B1**
(45) Date of publication and mention of the grant of the patent: **16.11.2016**
(21) Application number: 08874750.6
(22) Date of filing: 23.12.2008
(51) Int. Cl.: A61B 18/14, A61B 18/12, A61B 46/13, A61B 17/42, A61B 18/00, A61B 50/30

(54) **SYSTEM FOR ABLATIONAL TREATMENT OF UTERINE CERVICAL NEOPLASIA**
SYSTEM ZUR ABLATIONSBEHANDLUNG VON NEOPLASIEN IM GEBÄRMUTTERHALS
SYSTÈME POUR LE TRAITEMENT ABLATIF D'UNE NÉOPLASIE CERVICALE UTÉRINE

(30) Priority: 18.06.2008 US 73722 P; 13.11.2008 US 270373
(43) Date of publication of application: 30.03.2011
(73) Proprietor: Covidien LP, Mansfield, MA 02048 (US)
(72) Inventor: UTLEY, David, S., Sunnyvale CA 94085 (US); GERBERDING, Brent, C., Sunnyvale CA 94085 (US); TAIMISTO, Miriam, H., Sunnyvale CA 94085 (US); CHUNG, Winnie, Sunnyvale CA 94085 (US); MARLER, Jennifer, D., Sunnyvale CA 94085 (US); FILIMON, Viorica, Sunnyvale CA 94085 (US)
(74) Representative: Maschio, Antonio
(86) International application number: PCT/US2008/088209
(87) International publication number: WO 2009/154654

(56) References cited:
- WO-A2-2007/143281
- GB-A- 2 347 083
- GB-A- 2 434 545
- US-A- 5 160 334
- US-A- 5 403 310
- US-A- 5 840 077
- US-A- 5 938 694
- US-A- 5 951 550
- US-A1- 2002 161 363
- US-A1- 2005 149 013
- US-A1- 2005 171 525
- US-A1- 2006 135 956
- US-A1- 2007 100 335
- US-B2- 6 929 642

## Description

### FIELD OF THE INVENTION

The present invention relates generally to medical systems for treatment of the uterine cervix. More particularly, the invention is directed toward ablational treatment of uterine cervical epithelial disease such as cervical neoplasia.

### BACKGROUND OF THE INVENTION

Uterine cervical intraepithelial neoplasia (CIN) is a pre-cancerous condition of squamous epithelial cells on the surface of the cervix. This neoplastic change to the epithelium is caused by persistent infection with one of about 15 human papilloma virus (HPV) genotypes. In some patients, the neoplastic cells may progress in severity and extent to involve the entire thickness of the epithelium covering the cervix. Once the neoplastic cells invade the basement membrane of the epithelium, the disease is designated invasive cancer. Other risk factors for the development of cervical neoplasia include HIV infection, smoking, multiple sexual partners, and use of oral contraceptives.

Uterine cervical cancer is second only to breast cancer as the most common type of cancer in women worldwide. Between about 250,000 and 1 million American women are diagnosed with CIN and cervical cancer annually. Women in the 25 to 35 year age group are most likely to present with CIN, but it can develop in women both younger and older than that age group. If the condition is detected and treated early in the CIN stage, it is usually curable, thus precluding a progression to more advanced and invasive neoplastic stages (cancer) of the disease.

Various systems have been developed to classify (CIN) according to its severity and degree of involvement of the epithelium. In Europe, a grading system of CIN 1, 2, and 3 is used predominantly; in the U.S., a system of LSIL (low-grade intraepithelial lesions) and HSIL (high-grade intraepithelial lesion) is more commonly used. CIN1 and LSIL represent mild CIN and correspond to the early inflammatory reactions to HPV infection. This mild stage is not a predictor of cancer progression, is not an indication for treatment, and most cases resolve spontaneously. CIN 2 and CIN 3 correspond to HSIL, referring to a moderate or severe CIN. CIN2 is moderate neoplasia confined to the basal 2/3 of the epithelium. CIN3 is a severe neoplasia that spans more than 2/3 of the epithelium, and may involve the full epithelial thickness. In early scientific literature, CIN-2 and CIN-3 were referred to as carcinoma-in-situ (CIS), but this nomenclature has been abandoned. As cells of CIN-3 lesions accumulate serial oncogenetic abnormalities, some cells may penetrate through the basement membrane of the epithelium and invade the underlying stroma, at which point the lesion has become invasive cancer. Uterine cervical cancer staging systems, such as that of the International Federation of Gynecology and Obstetrics (IFGO) are then used to designate the state of the disease.

Available methods for treatment of CIN are directed toward destruction or excision of the abnormal epithelial cells. CIN-2 and CIN-3 lesions are typically targeted with ablative or excisional techniques to avert cancer development. Some very early invasive cancers may be treated in a similar manner. Ablation methods include cryoablation, fulguration with electrocautery, laser ablation, surgical excision with loop electrical excision procedure (LEEP), and laser or cold knife cervical conization. Ablation and excision result in comparable rates of clearance for CIN (80 - 90 %). Excision offers the advantage of providing a pathological specimen for histologic examination, but is also burdened by the disadvantage of increased surgical complications such as bleeding and cervical incompetence. The overall complication rate of 2 - 8% and a specific cervical stenosis complication incidence of 1 - 3% are comparable for ablative and excisional techniques.

US 2006/0135956 discloses a device for occluding a fallopian tube. The device comprises an RF applicator head including an electrode carrier with one or more bipolar electrodes thereon.

US 2005/0171525 discloses an ablation system comprising a catheter with a lumen, a conductive element disposed along the catheter and a balloon having an interior in fluid communication with the lumen of the catheter.

WO-A-2007/143281 discloses treatment of CIN using high-intensity focused ultrasound energy.

Improved methods of treatment of CIN and early invasive cancer would be highly desirable. An alternative modality, as provided by the ablation system of the invention, is that of an ablational approach that provides a high degree of ablation depth control and assurance of complete eradication of neoplasia without undue side effects.

### SUMMARY OF THE INVENTION

The present invention provides an ablation system for treating abnormal epithelial tissue of the uterine cervix according to claim 1.

Preferred embodiments are defined in the dependent claims. Embodiments, examples and aspects disclosed herein, but not falling under the scope of claim 1, do not form part of the invention. This applies in particular to the methods disclosed herein.

The support surface of the operative head may also be referred to as an energy-delivery surface or an electrode-bearing surface. The energy-delivery surface of the ablation device is substantially complementary to the proximal-facing surface of the cervix; and by this complementary fit, a therapeutically effective contact between the energy-delivery surface and the cervix can be achieved. With such a therapeutically effective contact, the delivery of energy, per methods summarized below, can be predictable and controlled, such that the depth of ablation into tissue, and the surface area boundaries of ablated tissue can be well controlled.

The ablation device includes a shaft that supports the operative head on a distal portion of the shaft, the shaft sized to be accommodated within the vagina and of sufficient length to reach the cervix from the vaginal entrance. The ablation device further includes a handle that supports the proximal portion of the shaft. The distal support surface may be substantially round in its frontal profile, and may assume various surface configurations, including being substantially flat, concave, or conical. Some embodiments of the support surface include a center post adapted to enter the cervical os, and thereby provide a stabilizing or orienting benefit that facilitates the seating of the distal face of the ablation probe head on the ectocervix. The center post may either support energy delivery elements, or it may be devoid of such elements.

Examples of radiofrequency delivery elements include one or more monopolar electrodes, one or more bipolar electrode pairs, a bipolar electrode array, electrodes circumferentially aligned on the support surface, electrodes on the support surface aligned axially with respect to the shaft, or electrode traces, which may be any of a press-fit design, an insert-molded design, a bondable design, a conductive-ink design, a flex-circuit design, or any combination of the above.

Embodiments of the energy delivery element of the ablation device include electrodes on the electrode-bearing surface that are typically spaced apart at intervals in the range of about 0.1 mm to about 4 mm. In some embodiments, however, the spacing may be less than 0.1 mm, and in some embodiments, the spacing may be wider, up to about 10 mm, for example. These latter wider spacing intervals provide flexibility in the device to allow ablation to deeper tissue depths. Embodiments of the energy delivery element include electrodes that have a width in the range of about 0.1 mm to about 4 mm.

The radiofrequency electrodes are configured into zones that are served by independently operable channels. Other embodiments include ones where the elements are configured into zones with different electrode densities. In some embodiments, the surface of the electrode-bearing support has a portion that is devoid of electrodes and one or more zones where electrodes are arranged on the support.

In some embodiments of the ablation device, the operative head includes a rollable sheath that is configured to unroll proximally to cover the shaft of the device. The operative head, itself, may be sterilized, along with the rollable sheath, such that when the sheath unrolls, it provides a sterile covering over the shaft and a protective barrier between the device and the patient's tissue. In some embodiments, the device may further include a speculum adapted to accommodate and secure the handle and shaft of the device therethrough.

The frontal profile of ectocervix of the uterine cervix lies at an angle to the longitudinal axis of the vagina, therefore embodiments of the ablation device may include features to optimize the engagement of the distal electrode-bearing surface of the ablational probe head. In some embodiments of the ablation device, a distal portion of the shaft includes a flexible portion configured to allow the distal support surface of the operative head to engage the cervix. In other embodiments of the ablation device, a distal portion of the shaft comprises an angled portion configured to allow the distal support surface of the operative head engage the cervix.

Some embodiments of the operative head (also referred to as an ablation probe head) the ablational device include means to stabilize therapeutic contact of the distal support surface with the cervix. Some embodiments, for example, include a clasping feature that actively engages a portion of the cervix, thereby stabilizing therapeutic contact, or applying pressure to secure such therapeutic contact. In other embodiments, the operative head includes a vacuum manifold that draws tissue and electrode-bearing surface of the device together. In still other embodiments, the operative head includes an expandable balloon that is insertable through the cervix and into the uterus such that upon expansion of the balloon, a pulling force is generated by the balloon that draws the electrode bearing surface of the operative head into therapeutic contact with the cervix.

The system may further include any one or more of a grounding pad, a foot pedal to control the generator, and a speculum adapted to accommodate and secure the handle and shaft of the device therethrough.

Embodiments of the system, by way of the configuration of the generator and the energy delivery elements, may be configured to deliver RF energy to the cervix at a power density that ranges between about 5 W/cm2 and about 150 W/ cm2, and to deliver RF energy to the cervix at an energy density that ranges between about 5 J/ cm2 and about 100 J/cm2. Embodiments of the system may be configured to deliver energy to the cervix in one or more pulses. Embodiments of the system may further be configured to receive feedback, and to use such feedback to terminate the energy delivery, such feedback being based on any of energy dose delivery, impedance within the cervix, temperature within the cervix, or time duration of energy delivery.

Also disclosed is a method for ablating abnormal tissue of the uterine cervix that makes use of the system and device, as summarized above. Thus, the method includes advancing an ablation device through the vagina toward the cervix, aligning an energy delivery element support surface conformably against a region of the cervix with abnormal tissue, and ablating the abnormal tissue with energy applied to the cervix from an energy delivery element on the energy delivery element support surface. Abnormal tissue ablated by this method may include neoplastic cervical tissue of any level of progression. The method may include visualizing the cervix prior to an ablational procedure to localize lesions, at a time during or in close proximity to the procedure to evaluate the immediate effect of ablational treatment, and/or at a later time, to evaluate the effectiveness of the ablation treatment. The method may also include expanding the vagina as a part of an ablation treatment, so as to facilitate access of the device to the cervix and to provide visual access of the site to the treating physician.

In some methods, ablating with energy includes delivering radiofrequency energy. And in various methods, delivering ablational energy, such as radiofrequency energy, includes controlling the delivery of energy such that the depth of cervical tissue ablation is controlled. Also, in some methods, delivering ablational energy, such as radiofrequency energy, includes controlling the delivery of energy such that the surface area that receives ablational energy is controlled.

With regard to controlling the depth of ablation within cervical tissue, focusing on regions that have been identified as having cancerous lesions, controlling the depth within cervical tissue to which ablation energy is delivered includes controlling the power density within a range that varies between about 5 W/cm² and about 150 W/ cm². Controlling the depth within cervical tissue to which ablation energy is delivered may also include controlling the energy density within a range that varies between about 5 J/ cm² and about 100 J/cm².

With further regard to controlling the depth of the ablation within cervical tissue, in some methods, the ablational energy is delivered from the surface of the cervical epithelium to a depth from about 0.1 mm to about 4 mm. In other methods, ablational energy is delivered from the surface of the tissue to a depth from about 0.2 mm to about 2 mm. In still other methods, ablational energy is delivered from the surface of the tissue to a depth from about 0.2 mm to about 1 mm. Regarding the deeper ranges of depth, for example, ablation to a level deeper than about 0.4 mm, these deeper ablations have such depth in order to ablate deeper lying regions of cervical epithelium, as for example, in the form of cervical crypts that become involved in a neoplastic process. In more specific regard to the ablation of crypts, controlling the depth of energy delivery includes delivering energy to a depth sufficient to ablate the deepest portion of a crypt.

During an ablation treatment procedure, the electrode-bearing surface of the operative head establishes an area of therapeutic contact with the cervix. Within that area of therapeutic contact, ablation energy may be delivered variably, in a zone-specific manner. Controlling the ablationally-treated surface area may occur by several approaches as well as a combination of such approaches. For example, in some embodiments, controlling the surface area subjected to ablation includes varying energy delivery according to concentric zone within the area of therapeutic contact. Controlling the surface area subjected to ablation may also include varying energy delivery according to a radial zone within the area of therapeutic contact. Thus, for example, treatment zones may be distributed concentrically as well as radially within the area of therapeutic contact.

The density of energy delivery may be varied within zones of the area of therapeutic contact both by having electrode density physically vary within zones of the electrode-bearing surface as well as by operably-controlling, at the generator level, the flow of energy to subsets of electrodes within zones. In some embodiments, the electrode-bearing support has a portion of its surface devoid of electrodes and a portion with electrodes arranged on the surface; in this embodiment, controlling the surface area of cervix included within the area of therapeutic contact includes positioning the operative head on the cervix such that the electrode-bearing zones of the distal surface of the device are adjacent to dysplastic or neoplastic target areas of the cervix.

In some methods, controlling the ablationally-treated surface area may include varying energy delivery, by way of delivering varying levels of energy to varying subsets of electrodes, according to concentric zone within the area of therapeutic contact. The method may further include varying energy delivery according to radial zone within the area of therapeutic contact.

The method may include deriving ablational energy to transmit from the operative head from an energy source in a manner that is controlled by a control system. In various embodiments of controlling the delivery of energy from the generator, such control may include controlling energy delivery so as to provide any of a specific power, duration of energy delivery, power density, energy, energy density, impedance, or tissue temperature.

The method may include visually evaluating the cervix to assess the location, size, and stage of dysplastic or neoplastic lesions. Such evaluation may occur prior to treatment, in which case location and size information may be used to plan the distribution of ablational energy from zones of the electrode-bearing surface during treatment. In other methods, visual evaluation of the cervix may occur during treatment, if, for example, energy is delivered multiple times, or visual evaluation may occur at various time intervals after the treatment, such as a time in close proximity to the treatment (days or weeks), or a later follow times (months or years).

### BRIEF DESCRIPTION OF THE DRAWINGS

**Figure 1** depicts an embodiment of a system used for ablation that includes the generator and disposable unit.
**Figure 2A** provides a perspective view of an embodiment of a cervical ablation device.
**Figure 2B** provides a perspective view of an embodiment of a cervical ablation device that is integrated within or fitted into a speculum.
**Figures 3A - 3C** provide views of a device that includes sheath feature built into the design of the ablation head to protect a reusable handle assembly. **Figure 3A** shows the sheath in a rolled configuration around the base of the ablation head. **Figure 3B** shows a detailed cross sectional view of the ablation head with the sheath in a rolled configuration. **Figure 3C** shows the device with the sheath unrolled, and covering the shaft and a portion of the handle of the device.
**Figure 4A** shows a cross sectional and partly rotated view of the vagina, cervix, and a portion of the uterus.
**Figure 4B** shows an ablation head in isolation (without the shaft) in position overlaying the cervix. The back of the ablation head includes a transparent window or physical opening at which an optical fiber may be positioned for visualization of the cervix.
**Figure 5A** provides a cross-sectional view of the larger anatomical context of human female anatomy.
**Figure 5B** shows an ablation device inserted through the vagina, with the ablation head positioned in therapeutic contact with the cervix. The distal portion of the shaft may include a fixed angle, or it may be flexible so as to allow passive engagement of the cervix at an appropriate angle.
**Figure 5C** shows an ablation device with a deployed protective sheath. This view may represent either the ablation head approaching the cervix, or being withdrawn from it.
**Figure 6A** is a schematic diagram of a histological cross section of uterine cervical epithelium with a left-to-right progressive depiction of the progression of a neoplastic lesion.
**Figure 6B** is a schematic diagram of a tissue cross section of uterine cervical wall and the base of the vagina, showing details associated in the vicinity of the cervical os, including cervical crypts.
**Figure 7** depicts an embodiment of a cervical epithelium ablation probe head with a flat electrode configuration.
**Figure 8** depicts an embodiment of a cervical epithelium ablation probe head with a concave electrode configuration.
**Figures 9A - 9F** depict various embodiments of a cervical epithelium ablation probe heads that vary in shape and electrode alignment. **Figure 9A** shows an embodiment of a cervical epithelium ablation probe head with a bowl-shaped concave electrode-bearing surface configuration and a centering element that is integral with the electrode.
**Figure 9B** shows an embodiment of a cervical epithelium ablation probe head with a bowl-shaped concave electrode-bearing surface configuration and a centering element that is not included as part of the electrode; the electrode configuration includes two concentrically-arranged monopolar electrodes.
**Figure 9C** shows an embodiment of a cervical epithelium ablation probe head with a shallow concave electrode-bearing surface configuration and a centering element that is integral with the electrode.
**Figure 9D** shows an embodiment of a cervical epithelium ablation probe head with a shallow concave electrode-bearing surface configuration and a centering element that is not included as part of the electrode.
**Figure 9E** shows of an embodiment of an ablation probe head with a shallow electrode-bearing surface and a projecting centering element, and electrode traces that align radially to the probe and extend up the centering element.
**Figure 9F** shows an embodiment of a conical cervical ablation probe head with electrode traces that align radially with respect to the probe.
**Figures 10A and 10B** show coordinate systems that can be used to align the ablation probe head against the cervix in preparation for applying ablation energy in a targeted manner. **Figure 10A** shows a system that maps the surface of the head with concentric coordinates. **Figure 10B** shows a system that maps the surface of the head with radial coordinates.
**Figures 11A - 11C** provide views of the frontal profile of therapeutic contact between the energy-delivering element support surface of the ablation probe head against a frontal view of the cervix. **Figure 11A** shows a contact area that substantially covers the entirety of the cervix.
**Figure 11B** shows a therapeutic contact area that occupies a concentric midsection of the cervix.
**Figure 11C** shows a therapeutic contact area that covers the central portion the cervix.
**Figures 12A - 12F** provide views of areas of ablation energy delivery within a larger area of therapeutic contact between the energy-delivering element support surface of the ablation probe head. **Figure 12A** shows an area of ablational energy delivery that occupies a concentric midsection of the area of therapeutic contact.
**Figure 12B** shows an area of ablational energy delivery that occupies an outer concentric section of the area of therapeutic contact.
**Figure 12C** shows an area of ablational energy delivery that occupies a central concentric section of the area of therapeutic contact.
**Figure 12D** shows an area of ablational energy delivery that occupies an arc of the area of therapeutic contact.
**Figure 12E** shows an area of ablational energy delivery that occupies an area that is located in a radial arc subsection and is concentrically midway between the center and the periphery of the area of therapeutic contact.
**Figure 12F** shows an area of ablational energy delivery that is similar to that of Figure 12E but occupies a wider circumferential portion of the concentric midsection of the area of therapeutic contact.
**Figures 13A -13G** show frontal profiles of embodiments of the electrode bearing surface in which a portion of the surface is devoid of electrodes and another portion includes electrodes arranged into zones. **Figure 13A** shows an electrode array or a monopolar electrode arranged into a radially-centered concentric zone on the electrode-bearing surface.
**Figure 13B** shows an electrode array arranged into a radially peripheral concentric zone on the electrode-bearing surface.
**Figure 13C** shows an electrode array arranged into a radially-centered concentric zone on the electrode-bearing surface.
**Figure 13D** shows an electrode array arranged into a fractional circumferential arc zone on the electrode-bearing surface.
**Figure 13E** shows an electrode array arranged into a large oval or lobular zone on the electrode-bearing surface.
**Figure 13F** shows an electrode array arranged into a large oval or lobular zone on the electrode-bearing surface.
**Figure 13G** shows a dual monopolar electrode array arranged into a radially-centered concentric zone on the electrode-bearing surface.
**Figure 14A** depicts an embodiment of a cervical epithelium ablation probe head in section, showing multiple electrode channels covering the electrode surface.
**Figure 14B** depicts an embodiment of a cervical epithelium probe head in section, showing multiple electrode channels with varying electrode trace spacing covering the electrode surface.
**Figures 15A -15E** depict various aspects of the method of therapeutic contact between the electrode bearing surface of the ablation probe head and the surface of the cervix. Figure **15A** shows the ablation probe head approaching a cervix as in an ablation procedure
**Figure 15B** shows the distal surface of the ablation probe head in contact with the cervical surface in an orientation such that the zone is adjacent to the lesion.
**Figure 15C** shows an ablation probe head that is smaller in circumference than that shown in **Figures 15A and 15B****,** but with an electrode zone that still aligns against the lesion.
**Figure 15D** shows still another variation in the shape of an electrode bearing surface which includes a center post penetrating the cervical os, and into the cervical canal, as seen, for example, in **Figures 9A - 9F****.**
**Figure 15E** another variation in the shape of an electrode bearing surface, as may be appropriate for a particular cervical morphology, or the location of a cancerous lesion in such a particular morphology.
**Figures 16A and 16B** depict an embodiment of a cervical epithelium ablation probe head showing different styles of electrode traces covering the surface of the ablation probe head. **Figure 16A** shows the various electrode embodiments as a surface view.
**Figure 16B** shows the electrode embodiments of **Figure 16A** in a cross sectional view, showing the configuration of the attachment to the ablation head surface.
**Figure 17** depicts several ways to provide tissue apposition to the electrode, including a vacuum manifold, a clasping or bracketing feature, and a balloon that is able to extend forward into the uterus.
**Figures 18A - 18C** depict alternative embodiments of a single-use ablation probe. Figure 18A shows a spiral-shaped electrode configuration.
**Figure 18B** shows a compressed sine wave-shaped electrode configuration.
**Figure 18C** shows a daisy-shaped electrode configuration.

### DETAILED DESCRIPTION OF THE INVENTION

Provided herein are embodiments of a system and methods for ablational treatment of epithelial tissue of the female urogenital and reproductive systems for treatment of disease, such as neoplasia of the uterine cervix epithelium (also known as cervical intraepithelial neoplasia) and early invasive neoplasia (cancer). Other exemplary conditions or diseases of the urogenital tract that may be treated by embodiments of the system and methods include vaginal intraepithelial neoplasia, endometriosis, radiation vaginitis, rectovaginal, vesicovaginal or ureterovaginal fistulas, and vaginal or cervical vascular malformations such as angiomata, arteriovenous malformations, or angiodysplasia.

With more specific regard to ablation by way of radiofrequency energy, systems and methods provided herein include features that allow for delivery of energy that is well controlled and substantially uniform with respect to a surface area focus and a tissue depth focus within targeted areas of cervical epithelial tissue. Such tissue target area control is provided by calibration of exemplary variables such as power, energy, time, electrode spacing, electrode width, electrode array design and pattern, configuration of the energy delivery element, and apposition force, as described further below. Uniformity of ablational depth that involves the diseased epithelial tissue is also desirable as it decreases the incidence of complications such as scarring, bleeding, pain, cervical incompetence, perforation (in some targets) and other complications that are associated with ablation that penetrates too deeply. With regard to the cervix as an exemplary target, avoidance of ablation to a greater than desired depth decreases the incidence of associated longer-term complications such as cervical incompetence, stenosis, bleeding, ulceration, and others. Uniform depth of ablation decreases the likelihood of a treatment being incomplete due to inadequate penetration of the ablation effect and incomplete eradiation of neoplastic cells. More generally, treatment to a uniform depth also minimizes collateral damage to healthy tissue within the local epithelium and nearby organs, thereby sparing insult to the healthy tissue and supporting a quicker and more effective healing in the wake of treatment of the desired target area.

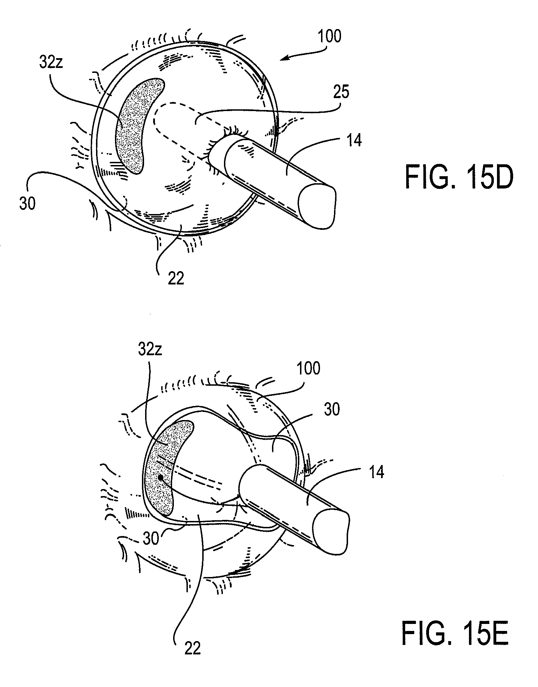

Ablational devices provided herein have a distally-directed energy-delivery element supporting surface that is substantially complementary to the ectocervical portion of the uterine cervix. The ablational surface of devices typically has a substantially circular frontal profile with either a substantially flat or concave surface; in some embodiments, the surface also includes a projecting center portion that serves to seat the device at the cervical target site. The frontal profile need not be perfectly round or symmetrical; it may, for example, be slightly elongate or oval in form (e.g., Figure 15E). In general the ablational surface is complementary to a tissue target site that can be described, from a proximal-facing front, as concave, funnel-shaped, or torus-shaped. The degree to which the periphery of ablational surface wraps around the peripheral surface of the torus represented by the cervix may vary. Further, the extent to which a central distally-projecting feature of the ablational surface engages the surface of the central canal portion of the cervix also may vary. In general, therapeutic contact between the ablational surface and cervical surface is provided by a physician operator applying an appropriate level of pressure during an ablational procedure. Some embodiments of the devices provided herein include features that can stabilize or secure the contact between the device and the cervix, such as a vacuum manifold. Some embodiments include a forward-pulling feature the draws the device into a secure contact with the cervix, such as an expandable balloon that can be inserted into the cervix, and upon expansion, provide a distally-drawing pressure on the ablational device to which it is anchored.

A particular exemplary embodiment of an ablation device to treat cervical cancer includes an ablation probe head (also referred to as an operative head) configured to approximate the size of the cervix and a centering post that is up to about 10 mm in length. The electrodes on a distal-facing electrode-bearing surface are typically arranged in a concentric bipolar electrode pattern. Electrodes are typically equally spaced apart at spacing intervals in the range of about 0.1 mm to about 4 mm and have a width in the range of about 0.1 mm to about 4 mm. Some embodiments of operative heads include a monopolar electrode configuration. A forward-projecting centering post (in the center of the electrode-bearing surface) may have about 5 mm of its base portion covered with an electrode array. Typically, radiofrequency (RF) energy is delivered by the device at a power density that ranges between about 5 W/cm² and about 150 W/ cm² and at an energy density that ranges between 5 J/ cm² and 100 J/cm². Energy can be delivered in a single pulse or in multiple pulses. The delivery and the termination of ablational energy delivery are controlled by a generator that is responsive to various feedback loops, and be can be energy dose-based, impedance-based, temperature-based, or time-based.

The invention and its features as generally described above, and as earlier summarized will now be further described in the context of particular embodiments and exemplary figures.

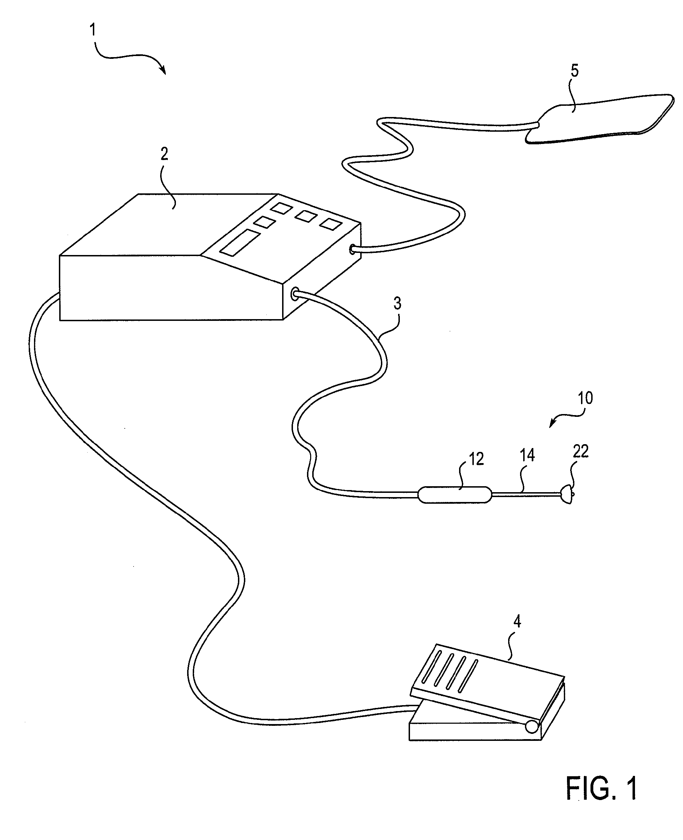

As shown in Figure 1, an exemplary ablation system 1 includes a radiofrequency generator 2 that has a cable 3 to deliver power, vacuum, fluid, light, and any other optional form of energy or service to an ablation device 10. The generator 2 also includes control features that govern the delivery of power, energy, and the duration of treatment to achieve the desired ablation results. An ablation device 10, as provided with the system, includes a handle 12, a shaft 14, and an ablation probe head (or operative head) 22 that is particularly adapted to access the cervix through the vagina, conformably engage the cervix, and deliver ablational energy to a target area on the cervix. The system may also include control features such as a foot pedal 4 for initiating delivery of ablational energy. The ablation probe head 22 may be an integral part of the ablation device **10,** or it may be a single-use element that plugs into the handle **12** and is disposed of after each use. In some embodiments, the ablation device **10** operates with electrodes in a bipolar mode. In other embodiments, the electrodes may be operated in a monopolar mode, with an electrical grounding pad **5** as part of the system **1.**

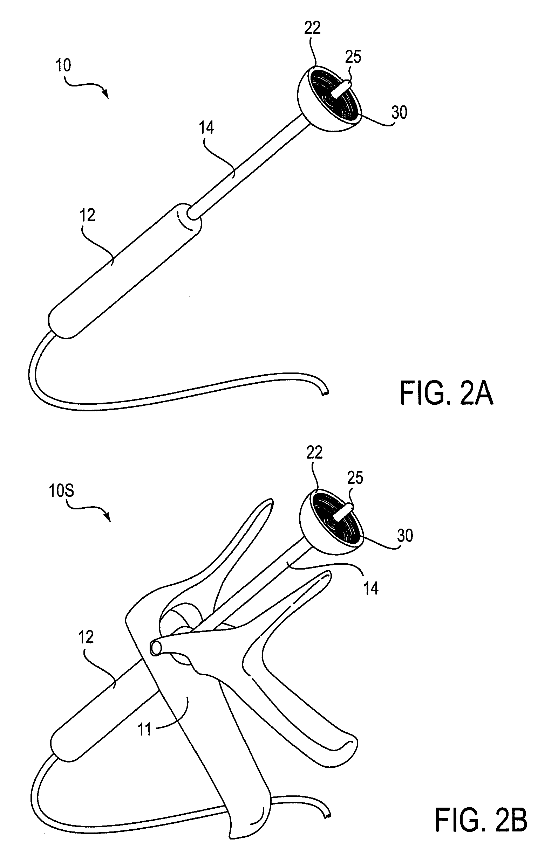

**Figure 2A** provides a perspective view of an embodiment of a cervical ablation device **10,** including a handle **12,** a shaft **14,** and an ablation head **22** with an electrode-bearing surface **30.** **Figure 2B** provides a perspective view of an embodiment of a cervical ablation device similar to that of **Figure 2A** except that the device **10,** as a whole, is supported within a speculum **11,** thereby forming a combination device **10S**. The function of the speculum is to facilitate access of the device through the vagina to enable contact of the distal support surface **30** of the ablation probe head against the uterine cervix. Some embodiments of device **10S** may be formed as integrated combination unit, whereby the shaft **14** and or the handle **12** of the device are unified with a speculum **11.** In other embodiments, the speculum itself may include a handle for the device. In other embodiments, the device **10** and the speculum **11** may be separate devices that are adapted and configured to be functionally joined.

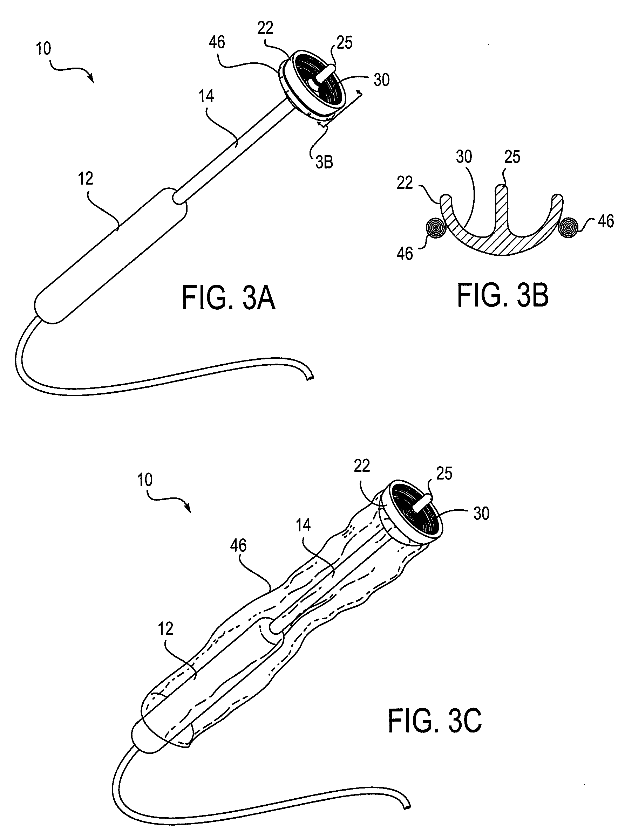

The ablation head **22** is typically sanitized or sterilized before use, or it may be a single-use sterile-packaged unit, as mentioned above. As such, the ablation probe head is typically a distinct unit that can be readily engaged or disengaged from the shaft **14.** Such engagement includes the physical attachment of the ablational probe to the shaft, but also of supply lines that convey energy to the ablation probe head. Further, some embodiments of the invention do include a unitary shaft-plus-ablation probe head configuration. The handle **12** and/or the shaft **14** of the device **10,** however is typically a more durable item than the ablation probe head, which although not necessarily sterile, is desirably cleanable, sanitized, and exposed to contamination as little as possible prior to use. In order to provide a level of sanitary protection to the handle or shaft, a condom-like protective sheath **46** can be included as part of the ablation head **22,** as shown in **Figure 3A** and **3B. Figure 3A** shows the condom feature in a rolled configuration, as it would be in a sterile or sanitary package. Upon attaching the ablation head to the handle assembly, the protective sleeve is unfurled as shown in the cross-sectional detail view provided by **Figure 3B** (electrodes not shown). The protective surface **46** may simply be left in place over the shaft, during an ablation procedure. In some embodiments, the protective sheath has one or more attachment features at its base which can be attached to complementary features at proximal portion of the shaft or a distal portion of the handle in order to prevent the sheath from being drawn distally during the procedure, or upon withdrawal of the device from the vagina after completion of the procedure.

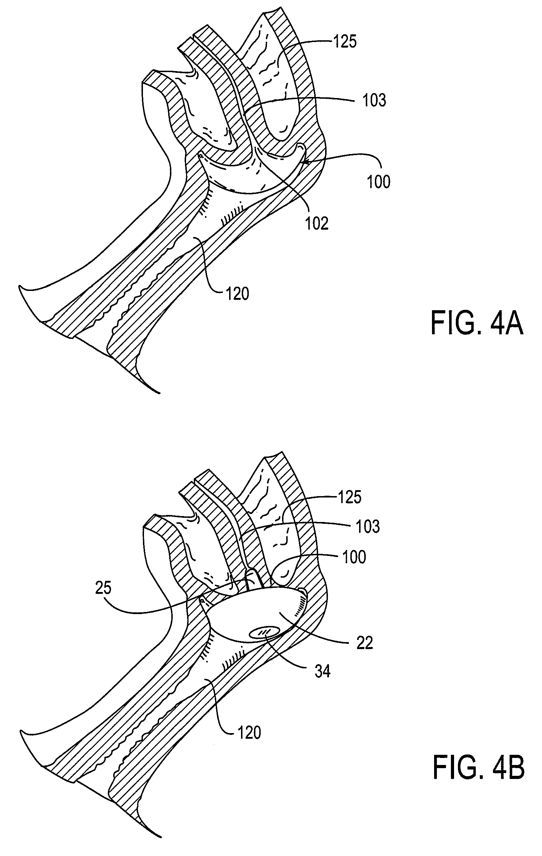

**Figure 4A** shows a cross sectional and partly rotated view of the vagina **120,** the cervix **100** (the thickened ectocervical portion is seen projecting into the vaginal canal), the cervical opening or os **102,** the cervical canal or cavity **103,** and a portion of the uterus **125.** **Figure 4B** shows an ablation head in isolation (without the shaft, for clarity with regard to the position of the ablation probe head **22**) in position overlaying the cervix **100.** It may be desirable prior to or during a cervical ablation procedure for the physician to visualize the cervix **100.** Accordingly, as in **Figure 4B****,** some embodiments of the ablation head **22** include a transparent window **34** through which a distally-directed light may be shone to illuminate the cervix or a particular target area. A light (not shown) may be directed into the ablation head via fiber optics, light-emitting diodes, or by other means to illuminate the cervical surface to aid with positioning and ablating.

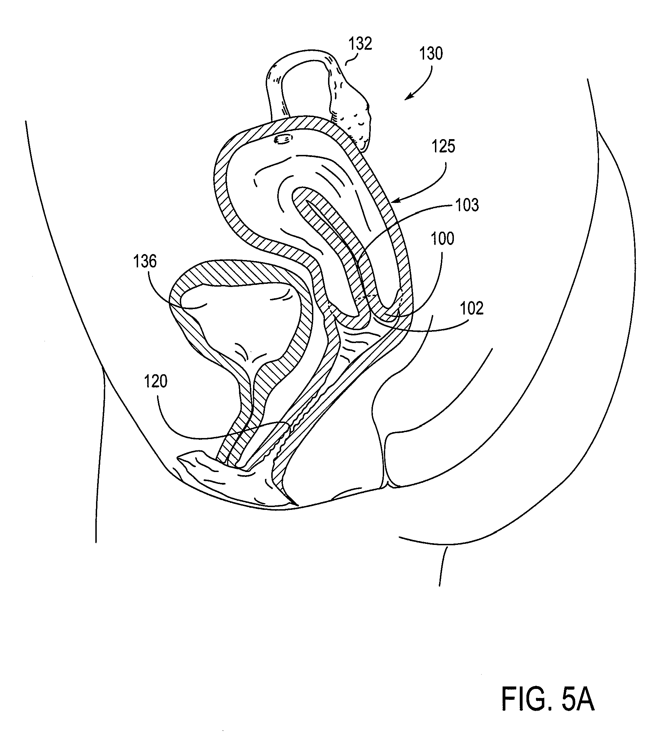

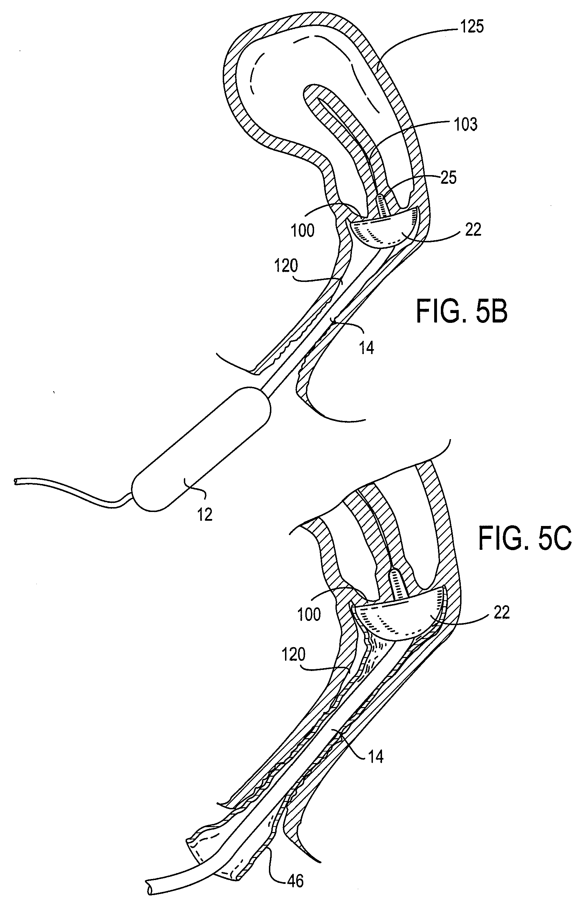

**Figure 5A** provides a cross-sectional view of the larger anatomical context of human female anatomy. **Figure 5B** shows an ablation device inserted through the vagina, with the ablation head positioned in therapeutic contact with the cervix. The distal portion of the shaft may include a fixed angle, or it may be flexible so as to allow passive engagement of the cervix at an appropriate angle. **Figure 5C** shows an ablation device with a deployed protective sheath **46.** This view may represent either the ablation head approaching the cervix, or being withdrawn from it.

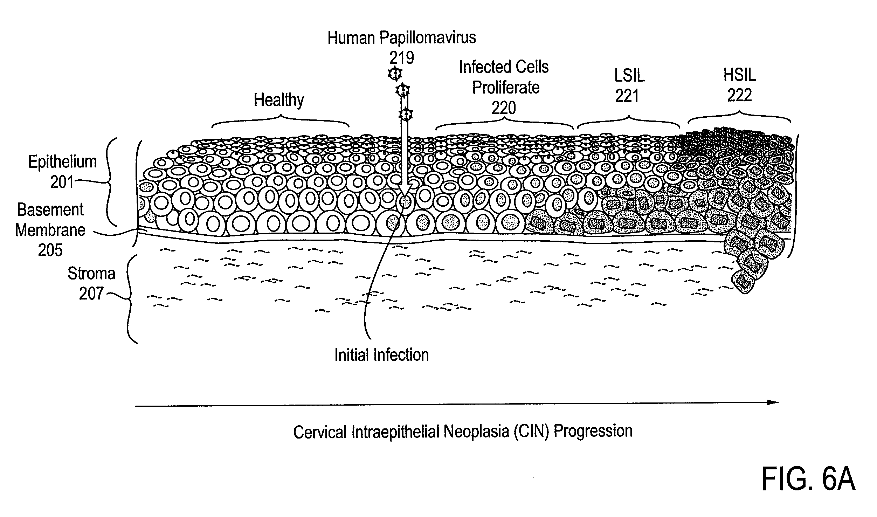

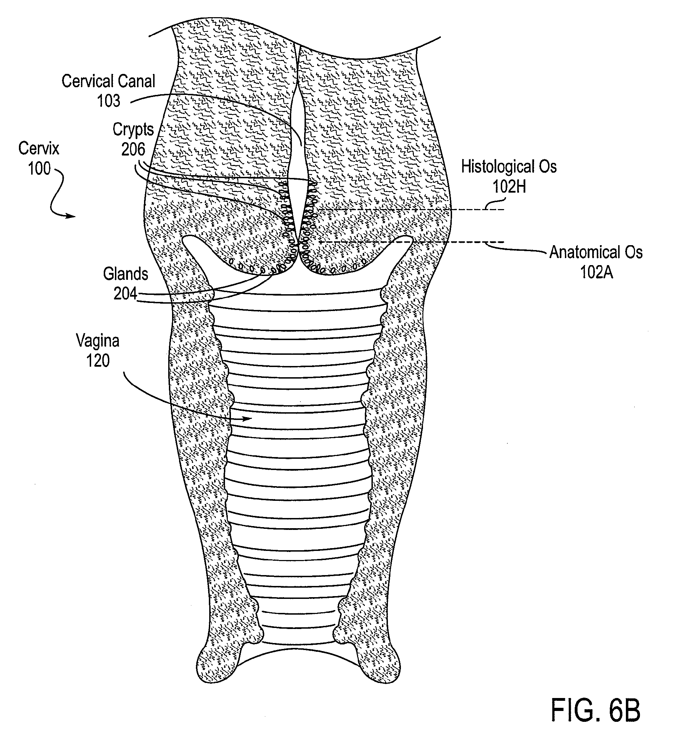

**Figure 6A** is a schematic diagram of a histological cross section of uterine cervical tissue 100, with a focus on cervical epithelium with a left-to-right progressive depiction of the progression of a neoplastic lesion. The epithelium **201** is the outermost layer and has a thickness of about 0.1 mm to 1.0 mm, with abnormal tissue having a thicker average depth. Cervical crypts may be present (see **Figure 6B**) and extend several millimeters below the main epithelial surface. The cervical epithelium **201** is bounded by an underlying basement membrane **205** and connective tissue stroma **207** below that. Therapeutic options may include ablation of the epithelium, or ablations of the epithelium and into greater tissue depths below (for example, to a maximal depth of about 5 mm below the epithelial surface) to ensure that neoplastic cells associated with cervical crypts are ablated as well.

As mentioned, **Figure 6A** includes a left-to-right chronological schematic representation of the progression of cancerous lesions on the cervix. According to present concepts of the etiology of a lesion, it begins with infection of a cell or a population of cells with the human papilloma virus (HPV) (**219**). Epithelial cells originate from a stem cell population that is located at the base of the epithelium, adjacent to basement membrane **205**; from there, the cells undergo differentiation into mature epithelial cells and they are moved upward to the epithelial surface by the proliferation of cells beneath them. HPV-infected cells initially appear as dysplastic or neoplastic cells **220** with a histologically-apparent abnormal form, and they become progressively less differentiated in comparison to their normal epithelial cell counterparts. As these cells proliferate and progress in their cancerous course, they become increasing dominant in their region and can be recognized as a low grade intraepithelial lesion (LSIL) **221.** Eventually, the population comes to occupy the full depth of the epithelial layer and by such dominance and state of progression by recognized as a high grade intraepithelial lesion (HSIL) **222,** extending from the basement membrane upward to the epithelial surface. As shown on the far right of **Figure 6****,** in a still further progressive state, cells of the cancerous lesion can migrate distally through the basement membrane into the stromal layer, at which point the cancer is recognized as an invasive carcinoma.

**Figure 6B** is a schematic frontal view of a tissue cross section of uterine cervical wall **100** as it joins the base of the vagina **120,** showing details of anatomical features in the vicinity of the cervical os The cervical os **102** is the opening within the ectocervical portion of the cervix (projecting into the base of the vagina) which provides an anatomical passageway between the vagina and the uterus. The os **102** is more specifically characterized in terms of the anatomical site where the opening is the tightest (the anatomical os **102A**) and the histological os site **102H** where the squamous epithelium **201** of the ectocervix changes to a columnar epithelium **202** that continues to line the cervical canal **103** into the uterus. While the location of anatomical os remains fixed, the histological site or squamocolumnar junction, moves over the course of the female's state of sexual maturity and age, *i.e*., the squamous cell population migrates into the anatomical region formerly occupied by columnar cells. The area within which this junction moves is known as the transition zone. Prior to sexual maturity, the histological os lies external to the anatomical os, with sexual maturity and advancing age, the histological os moves inwardly, passing the anatomical os, and into the cervical canal. **Figure 6B** depicts a cervix of a woman who is sexually mature, with the histological os **102H** deeper within the cervix than the anatomical os **102A.**

Underlying both the squamous epithelium outside the histological os and the columnar epithelium internal to the histological os are mucous-secreting glands **204** which increasingly enlarge into crypts **205** as they are distributed from the external periphery of the cervix inward through the os. These crypts open onto the epithelial surface and extend into cervical tissue to a depth in the range of about 4 - 5 mm. CIN lesions are initiated in the squamous cell region of the cervix, and thus also occupy the transition zone of the cervix, as described above. Further, the neoplastic lesions tend to involve the crypts **205,** and thus neoplastic cells can be located at the depths associated with the depth of the crypts (*i.e*., as deep as 5 mm). Embodiments of the method of the invention, as supported by embodiments of the inventive device (*e*.*g*., variable energy delivery parameters and variable widths of electrodes) are adjustable to vary the depth of ablation appropriately to the lesion. Thus, ablation to a depth corresponding to the range of the depth of the epithelium, in the range of 0.5 mm to 1 mm is a common implementation of the method. However, when cervical crypts are involved, ablation may occur to such corresponding depths, extending to a depth that reaches to the deepest portion of a crypt, or a depth of about 5 mm in most cases.

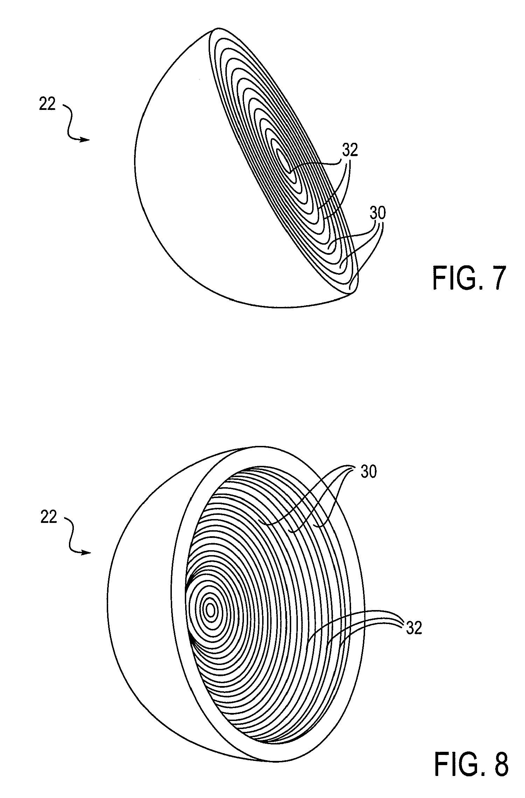

As shown in **Figures 7 - 9F****,** various embodiments of the ablation probe head geometry are possible. **Figure 7** shows an ablation head probe **22** with an electrode-bearing surface **30** that is flat. Such an embodiment may be appropriate for use when the targeted area of cervical dysplastic tissue is relatively small or narrow, and on the frontal surface of the cervix. **Figure 8** shows a cervical ablation probe head **22** with a concave electrode surface **30.** The concavity of the surface is adapted to conform to the frontal facing aspect of the cervix, and may vary in the degree of its concavity in various embodiments in order to be appropriate for the range of anatomical variation within the patient population. More specifically, the concave surface of the ablation probe head **22** is adapted to conform to the outer convex aspect of the ectocervix, also known as the *portio vaginalis*, the portion of the cervix that projects into the vaginal canal. Embodiments of this type, thus, are also generally adapted to be directed to sites of cervical neoplasia on the outward facing surface of the ectocervix. Electrode-bearing surfaces are indicated by a label **30** and electrodes of any type are indicated by a label **32** throughout.

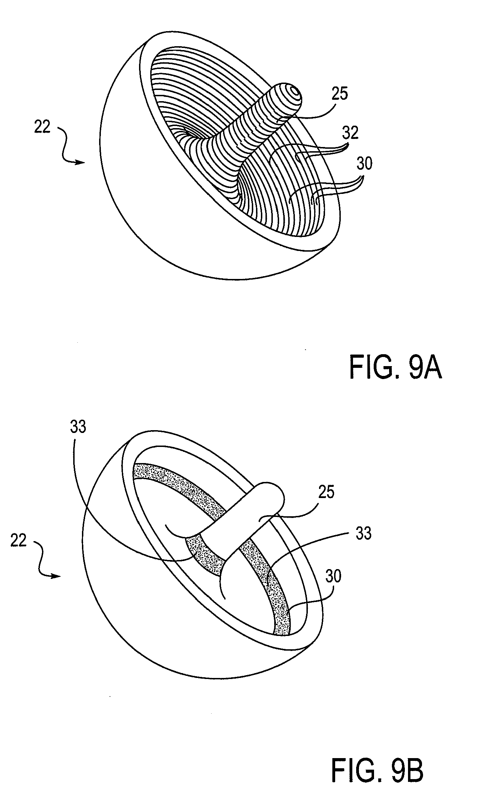

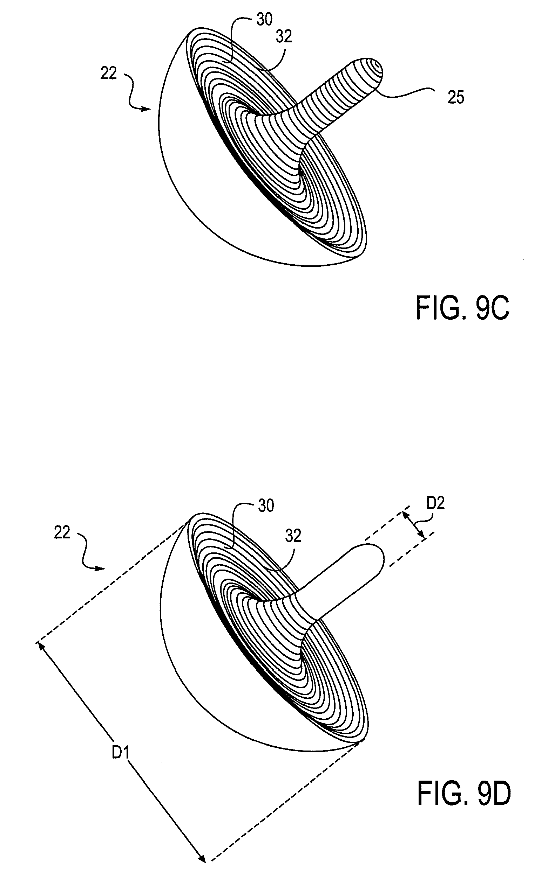

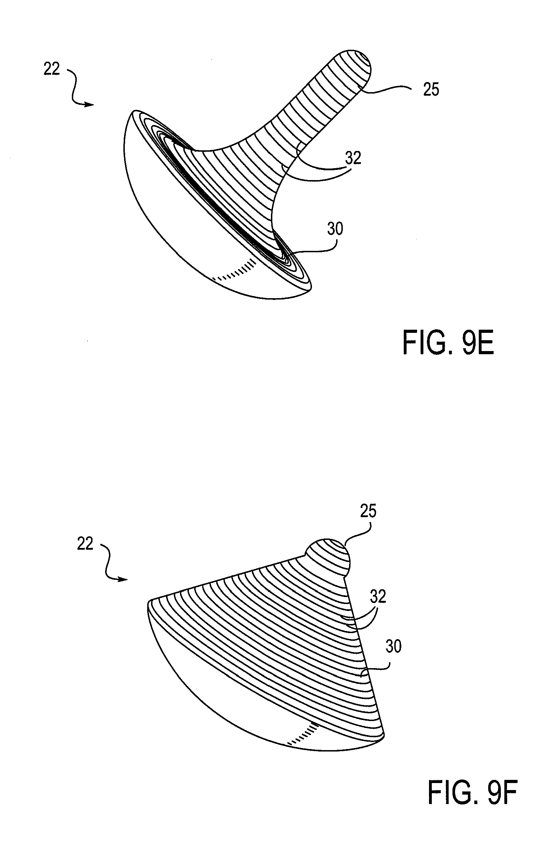

Embodiments of cervical ablation head probes that include a center post feature **25** are shown in **Figures 9A - 9F****.** This feature is generally adapted to facilitate the seating and centering of the ablation head **22** as it engages the cervix **100.** In some embodiments **(****Figures 9A****,** **9C****, and** **9E - 9F)** a center post **25** is generally contiguous with the electrode-bearing **30** aspect of the ablation probe 22. In these embodiments, the center post **25** may be directed toward the ablation of target areas on the interior aspect of the cervical os. In other embodiments **(****Figures 9B** **and** **9D****),** a center post **25** does not bear electrodes on its surface, and the structure simply serves a physically stabilizing purpose. These embodiments may be appropriate for treating cases of cervical dysplasia when it has been determined that the interior of the cervical os is substantially free of dysplastic or neoplastic cells.

**Figure 9A** shows an embodiment of the ablation probe head with a bowl-shaped or concave electrode configuration and a centering element **25** that is also part of the electrode bearing surface 30. Embodiments of the centering element **25** are generally adapted (physically, conformationally) to engage the cervical os and, in some embodiments as in **Figure 9A****,** to provide ablation inside the cervical os. The depth of the ablation into the cervical os **102** or cervical cavity **103** may be varied by changing the length of the centering element **25** and electrode-bearing surface thereon. **Figure 9B** shows an embodiment of the ablation probe head **22** with a bowl-shaped concave electrode surface **30** configuration and a centering element **25** that is not part of the electrode. The centering element **25** in this embodiment is only designed to engage the cervical os for the purpose of centering the device. The device embodiments **22** shown in **Figures 9A and 9B** both have a relatively deep concave aspect to their electrode-bearing surface **30,** and are thus adapted to be able to engage a relatively large portion of the ectocervix.

**Figure 9B** shows an embodiment of a cervical epithelium ablation probe head with a bowl-shaped concave electrode-bearing surface configuration and a centering element that is not included as part of the electrode. The electrode configuration includes two concentrically-arranged monopolar electrodes **33** on the surface of the electrode-bearing surface **30.** The monopolar electrodes may be operated independently; in embodiments of a method by which to use this type of electrode, one monopolar electrode may be operated while the other is not activated.

**Figure 9C** shows an embodiment of the ablation probe head with a shallow concave electrode configuration **30** and a relatively long centering element **25** that is also part of the electrode. The shallow concavity is designed to ablate less area of the cervix. The device embodiments **22** shown in **Figures 9C** - **9E****,** in particular all have a relatively shallow concave aspect to their electrode-bearing surface **30,** and are thus adapted to engage a relatively small portion of the ectocervix. **Figure 9D** shows an embodiment of the ablation probe head **22** with a shallow concave electrode-bearing surface **30** and a centering element **25** that is not part of the electrode. The shallow concavity is designed to ablate small area of the cervix. As shown in **Figure 9D****,** the diameter **D1** of the ablation probe head **22** is in the range of 10 mm to 35 mm and the diameter **D2** of an optional centering feature **25** is in the range of 1 mm to 10 mm.

**Figure 9E** shows an embodiment of the ablation probe head **22** with a relatively shallow concave electrode-bearing surface **30** and a relatively long center post **25,** which is adapted to ablate a relatively small surface area of the ectocervix and a relatively deep aspect of the interior central aspect of the cervix. **Figure 9F** shows an embodiment of the ablation probe head with a conical or tapered electrode-bearing surface **30** and a center post **25** of relatively minimal length.

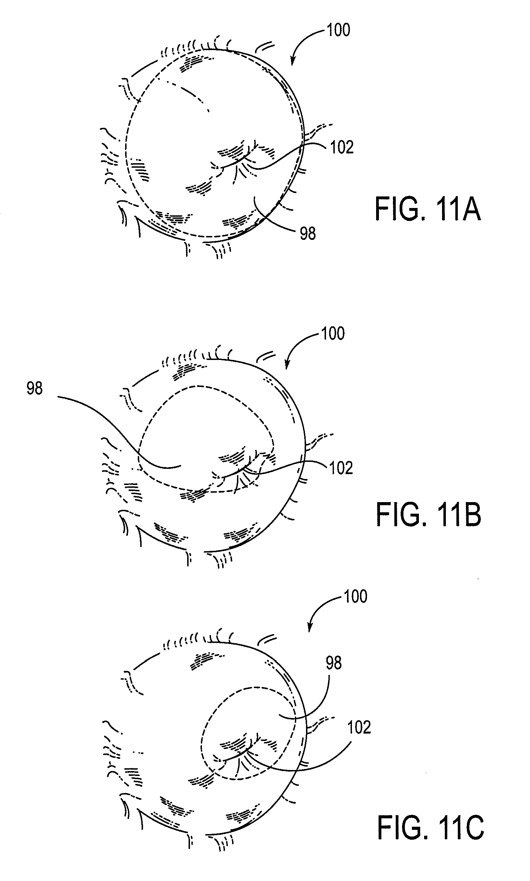

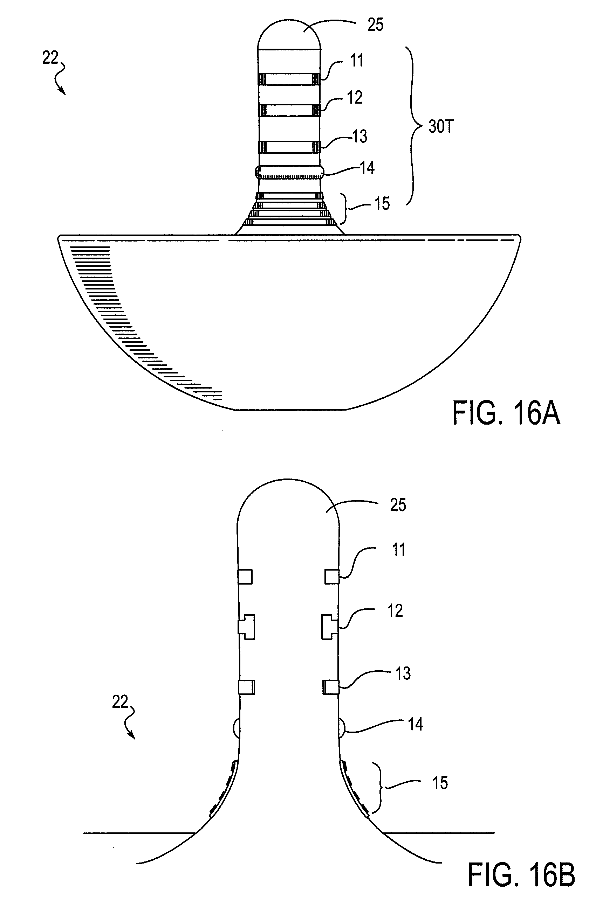

As shown by embodiments of the device in **Figures 7 - 9F****,** the distal-facing electrode support surface **30** of the ablation probe head **22** may be considered to define the boundaries of a therapeutic contact area between the device and the ectocervical surface when the device is brought into contact with the cervix. (The area of therapeutic contact **98** is described further below and depicted in **Figures 11A** **-** 11C.) The contact between this surface and the epithelial surface of the cervix can be therapeutically optimized by various approaches, such as those described below and in the context of **Figure 16****.** This contact area may vary in the degree to which it covers the available cervical or ectocervical surface **100.** In general distal-facing support surfaces that are substantially flat **(****Figure 7****)** have a smaller therapeutic contact area that those which are concave **(****Figures 8****,** **9A - 9D).** Those with a relatively shallow concave area **(****Figures 9C and 9D****)** have a smaller total contact area than those with a deeper concave surface **(****Figures 8****,** **9A, 9B****).** Also, in general, distal-facing contact surfaces with a center post that includes energy-delivery elements on the center-post **(****Figures 9A****,** **9C****,** **9E, and 9F****),** provide a larger therapeutic contact area than embodiments without a center post **(****Figures 9B** **and** **9D****).**

A particular embodiment of an ablation device includes a probe head configured to approximate the size of the cervix and have a centering post that is less than 10 mm in length. The electrode is typically arranged in a concentric bipolar electrode pattern with electrodes equally spaced apart at spacing intervals in the range of about 0.1 mm to about 4 mm. Each electrode has a width in the range of about 0.1 mm to about 4 mm. A forward-projecting centering post (in the center of the electrode-bearing surface) has about 5 mm of its proximal length covered with electrode array. Typically, radiofrequency (RF) energy is delivered at power density that ranges between about 5 W/cm² and about 150 W/ cm² and at an energy density that ranges between 5 J/ cm² and 100 J/cm². Energy can be delivered in a single pulse or in multiple pulses. Termination of delivery can be energy dose based, impedance based, temperature based, or time based.

Embodiments of the invention may include any of the variations described as examples provided herein, as well as any embodiment that combines features from any embodiment described herein. Some embodiments of the invention may take the form of a kit which includes components such as a radiofrequency energy generator, a grounding pad (for use in devices that have monopolar electrodes), a foot pedal, a cable for the device, a handle fitted with a shaft, and one or more ablation probe head variations as described herein. The ablation probe head and the shaft are mutually configured such that the shaft and the varied ablation probe heads have common mutually-engageable connections. In some embodiments of the method, ablation probe heads may be selected with a high degree of specificity such that they fit particular features of the patient's cervical anatomy or the cancerous lesions. In other embodiments of the method, it may be appropriate to use an ablation probe embodiment with a single overall physical size and shape, such embodiment having a one size fits all character that is broadly fitting of a large segment of the patient population.

Even with a common shape, the embodiment may vary in terms of the arrangement of electrodes on the electrode-bearing surface. Thus, even with a common size, the device can still be patient-tailored and lesion-tailored by the variation in electrode patterns (per embodiments of the inventive device), and by the handling and operation of the device (per embodiments of the methods of the invention). Such a one size embodiment, may, for example, include an embodiment of a general form such as that shown in **Figure 9D****,** with a center post that hosts electrodes at the base of the center post and extending forward to a point about 5 mm from the bottom of the center post. The electrode array on the electrode-bearing surface may vary, such that widths of the concentrically arranged array may be, for example, about 10 mm, 15 mm, 20 mm, or 25 mm.

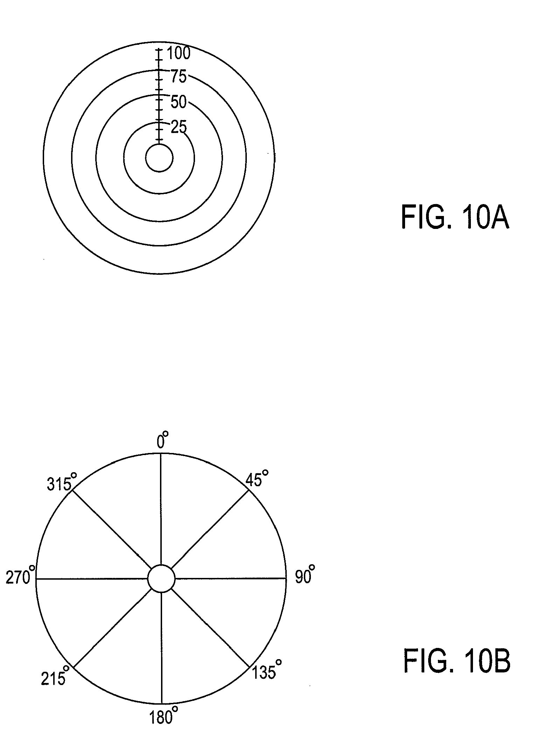

Cervical intraepithelial neoplastic lesions can be visualized on the cervix by several methods well known and practiced by gynecological physicians, and thus their location can be mapped on a coordinate system which can also be applied to the electrode-bearing surface **30** of an ablation probe head. Thus, by having the electrode-bearing surface make contact with the cervix in a known orientation, the position of cervical lesions can be located on the adjacent electrode-bearing surface of the probe head. **Figures 10A and 10B** show coordinate systems that can be used to align the ablation probe head against the cervix in preparation for applying ablation energy in a targeted manner. **Figure 10A** shows a system that maps the surface of the head with concentric coordinates. **Figure 10B** shows a system that maps the surface of the head with radial coordinates. In typical embodiments of a cervical ablation device, the radial orientation of the ablation probe head, and consequently the electrode bearing surface is fixed relative to the handle by which the physician manipulate the device. Thus, the physician, by the position of the handle, is generally aware of the orientation of the device with respect to the cervix, and of course, is aware of the position of cervical lesions from prior observation. For finer degrees of orientation, in some embodiments, a transparent window **34 (****Figure 4B****)** allows visualization of the surface of the cervix through the device. In some embodiments, the substantial entirety of the electrode bearing surface may be formed from a transparent material. Further, as an aid to orientation, the backing or proximal facing portion of the ablation probe may have one or more radially-arranged markings that can orient the physician operator. In general, visualization is considerably benefited by the use of a speculum **(****Figure 2B****)** that provides visual and illuminating access to the cervix.

**Figures 11A** - **11C** provide views of the frontal profile of an area of therapeutic contact **98** on a cervical surface **100** by the energy-delivering element support surface **30** of an ablation probe head **22** against a frontal view of the cervix **100** with the cervical os **102** in the center. A "therapeutic contact" or "therapeutically effective contact" between the electrode-bearing surface **30** and the cervical tissue surface **100** refers to a complete or substantially-complete contact between all or a portion of a cervix on the tissue surface by all or a portion of the electrode-bearing surface such that energy delivery from the electrode bearing surface to the cervical tissue surface is consistent and uniform. Such consistency and uniformity generally contributes to the predictability of ablation energy in terms of the depth in tissue to which an ablation effect is achieved. The electrode-bearing surface **30** of the ablation probe head 22, as described elsewhere, is substantially complementary to the proximally-exposed surfaces of the cervix; this conformational feature of the electrode-bearing surface provides particular functional benefit to the ablation probe head in that it supports such therapeutically-effective contact. **Figure 11A** shows a therapeutic contact area **98** that substantially covers the entirety of the cervix. **Figure 11B** shows a therapeutic contact area **98** that occupies a concentric midsection of the cervix. **Figure 11C** shows a contact area that covers the central portion the cervix.

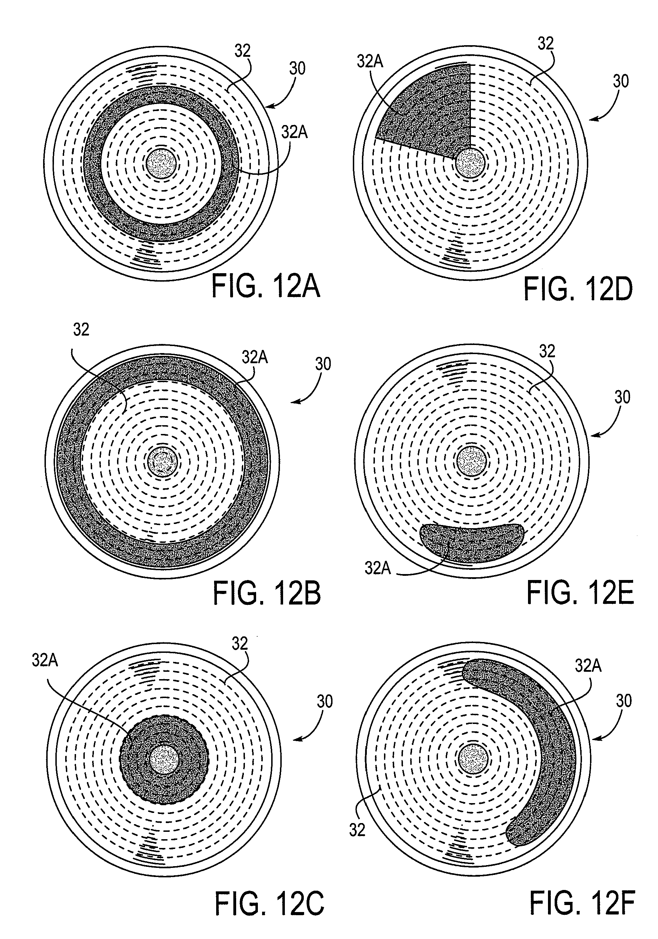

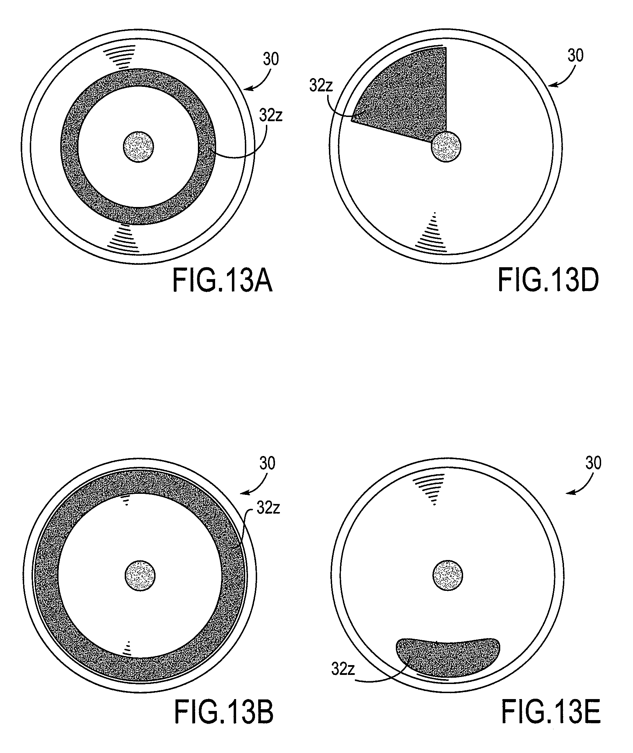

**Figures 12A** - **12F** provide frontal views of the electrode-bearing surface **30** of an operative head that is substantially covered by an electrode array **32** (depicted as concentric circles of dotted lines). The electrode array coverage may be selectively activated by various approaches (see below) so as to form zones of active electrodes **32A,** which when in contact with a cervical surface and activated during a treatment (see **Figures 13A -13F**) form an ablational zone within the larger area of therapeutic contact on the cervical surface (see the area of therapeutic contact **98** in **Figures 11A - 11C**). **Figure 12A** shows a zone of ablational energy delivery **32A** that occupies a concentric midsection of the area of electrode bearing surface **30.** **Figure 12B** shows a zone of ablational energy delivery **32A** that occupies an outer concentric section of the area of electrode bearing surface **30.** **Figure 12C** shows a zone of ablational delivery **32A** that occupies a central concentric section of electrode bearing surface **30.** **Figure 12D** shows a zone of energy delivery **32A** that occupies an arc of electrode bearing surface **30.** **Figure 12E** shows a zone of ablational energy delivery **32A** that occupies an area that is located in an arc subsection of the circumference that is concentrically midway between the center and the periphery of electrode bearing surface **30.** **Figure 12F** shows a zone of ablational energy delivery **32A** that is similar to that of **Figure 12E** but occupies a wider circumferential portion of the concentric midsection of the area of electrode bearing surface **30.**

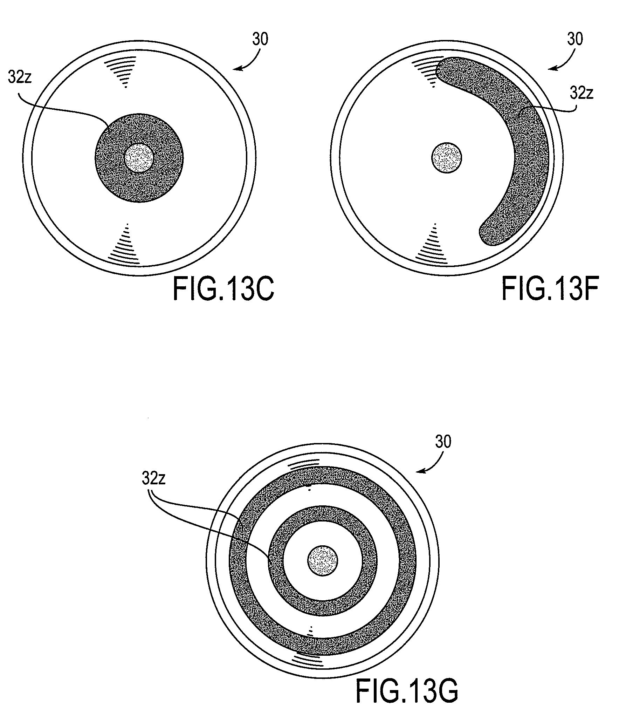

**Figures 13A -13G** shows frontal profiles of embodiments of the electrode bearing surface **30** of an ablation probe head **22** in which a portion of the surface is devoid of electrodes and another portion includes electrodes arranged into zones **32Z.** **Figure 13A** shows an electrode array arranged into a radially-centered concentric zone **32Z** on the electrode-bearing surface. **Figure 13B** shows an electrode array arranged into a radially peripheral concentric zone **32Z** on the electrode-bearing surface. **Figure 13C** shows an electrode array arranged into a radially-central concentric zone**32Z** on the electrode-bearing surface. **Figure 13D** shows an electrode array arranged into a fractional circumferential arc zone **32Z** on the electrode-bearing surface. **Figure 13E** shows an electrode array arranged into a large oval or lobular zone **32Z** on the electrode-bearing surface. **Figure 13F** shows an electrode array arranged into a large oval or lobular zone **32Z** on the electrode-bearing surface. **Figure 13G** shows two electrode arrays arranged into radially-centered concentric zone **32Z** on the electrode-bearing surface.

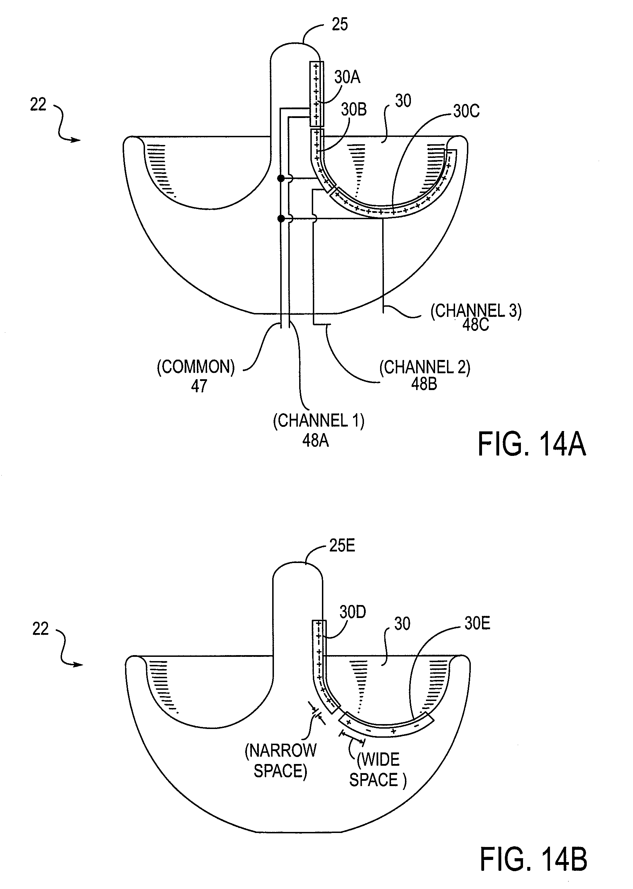

**Figure 14A** is a drawing of the ablation probe head **22** in section that shows multiple electrode channels supplying current to various zones of an electrode-bearing surface **30.** In a typical bipolar electrode embodiment, there may be a common channel **47,** and/or one or more active channels, such as **48A, 48B, or 48C** serving the electrode-bearing surface **30** in order to independently control the operation of electrode zones (as seen, for example, in **Figures 12A - 12F****.** These zones are distributed across the electrode-bearing surface, in accordance with the distribution of lesions on the surface of a cervix. As shown in **Figure 14A****,** for example, electrode-bearing surface zones **30A, 30B,** and **30C** are each served by separate respective multiple channels **48A, B, and C.**

**Figure 14B** shows an ablation probe head 22 in cross section that shows electrodes in separate zones that have different electrode spacing. In this example, electrode array **30D** is arranged with narrowly-spaced electrodes, and electrode array **30E** is arranged with widely-spaced electrodes. These zones may be served by either by a single electrode channel or by multiple channels. The differing electrode spacing allows further customization of the ablation characteristics about the surface of the cervix. Electrode spacing may vary between 0.25mm up to 10mm to achieve the desired depth of ablation. In general, other parameters being equal, widely-spaced electrodes form deeper ablation areas in tissue, and narrowly-spaced electrodes form shallower ablation areas in tissue.

By the approaches shown in **Figures 14A and 14B****,** or by a combination of such approaches, different ablation energy delivery operating parameters may be applied to the delivery of energy from separate zones within an electrode-bearing surface **30** of a cervical ablation device. The independently operable zones depicted in **Figures 10** **and** **11** are each merely examples. It can be understood that the electrode-bearing surface **30** of a cervical ablation probe head 22 may be configured into a large number of patterns in order to provide a high degree of flexibility regarding the distribution and size of dysplastic lesions on the surface of a cervix. Such flexibility coupled with images or visualization of the cervix enables the delivery of highly individualized ablation therapy for cervical intraepithelial neoplasia or early invasive neoplasia. In exemplary operations, the amount of energy delivered through electrode-bearing zones may vary, in some method embodiments, zones may be operated at full energy-delivery capacity, while no energy at all is delivered through other zones. In other examples, the duration of energy delivery time may vary from zone to zone.

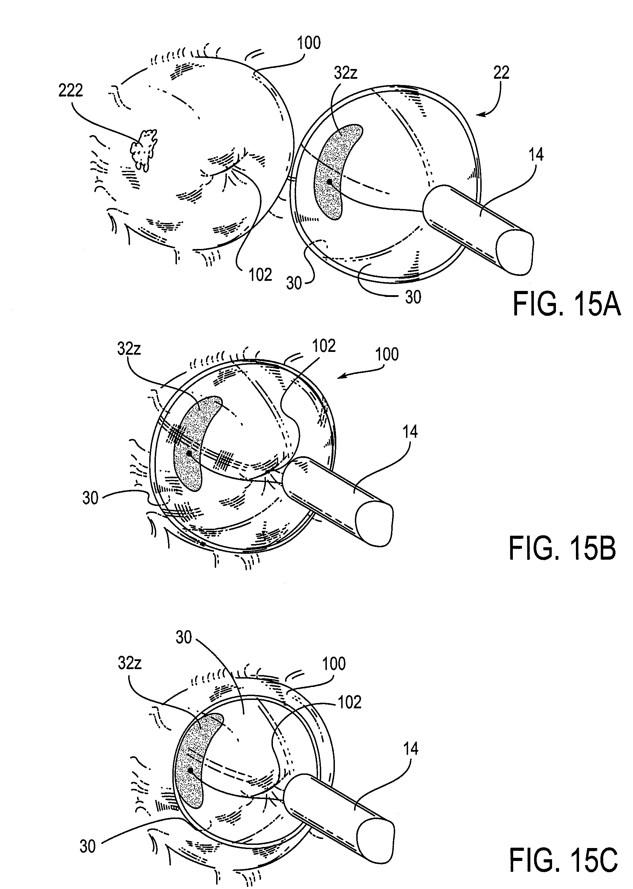

**Figures 15A -15E** depict various aspects of the nature of therapeutic contact between the electrode bearing surface **30** of an ablation probe head **22** and the surface of the cervix **100.** **Figure 15A** shows the ablation probe head approaching a cervix as in an ablation procedure. **Figure 15B** shows the ablation probe head **22** after it has made an effective therapeutic contact with the cervical surface. It can be seen that the electrode-bearing surface that includes a localized zone of electrodes **32Z** which is oriented such that it will be aligned with a cervical cancer lesion **222.** This alignment is made possible by earlier visual observations of the cervix that allowed mapping of the location of the targeted lesion, as, for example, by making use of a system of coordinates as shown in **Figures 10A and 10B****,** as well as by tracking the radial orientation of ablation probe head with a complementary coordinate system. In one embodiment, the method would make use of an ablation probe head with an electrode zone **32Z** that matches the size and location of the lesion **222,** as for example is the case with the electrode-bearing surface depicted in **Figure 13E** **or** **Figure 13F****.** In an other embodiment of the method, an electrode-bearing surface that is substantially covered with an electrode array, such as those shown in **Figures 12A - 12F** could be used, and in addressing such a lesion **222** as shown in **Figure 15****,** an electrode activation pattern such as that shown in **Figure 12E** or **Figure 12F** could be appropriately applied. In **Figure 15B****,** the distal surface **30** of the ablation probe head has been brought into contact with the cervical surface **100** in an orientation such that the zone **32Z** is adjacent to the lesion **222** by a physician who is manipulating the device.

**Figure 15C** shows an ablation probe head **22** that is smaller in circumference than that shown in **Figures 15A and 15B****,** but with an electrode zone **32Z** that still aligns against the lesion **222.** This figure exemplifies flexibility in the choice of the configuration of an ablation probe head and electrode-bearing surface to achieve a therapeutically effective contact between an electrode zone and a lesion. **Figure 15D** shows still another variation in the shape of an electrode bearing surface which includes a center post, as seen, for example, in **Figures 9A - 9F****.** **Figure 15E** shows still another variation in the shape of an electrode bearing surface, as may be appropriate for a particular cervical morphology, or the location of a cancerous lesion in such a particular morphology.

**Figure 16A** shows a view of a cervical ablation probe head **22** with different exemplary styles of electrode traces **30T** arranged on the center post **25.** **Figure 16A** provides a surface view, while **Figure** 16B shows a cross-sectional schematic view of ablation head **22** to illustrate details of the form of attachment to the ablation probe head. One embodiment for electrode trace attachment is of a press-fit trace design **11.** In this design, the electrode traces are pressed into the surface of the electrode head. Depending on the needs of the design, the surface of the electrode trace may lie above the surface, flush with the surface, or below the surface of the probe head.

Another electrode trace embodiment is one in which the electrode that is insert-molded **12.** In this design, the electrode traces are placed into a mold and the head material is molded around them. One advantage of this design is that the electrode may have securement features, such as a keyway shape, to prevent the electrode trace from detaching from the head unless the head material is physically deformed.

Another embodiment for electrode trace attachment is an electrode that is bonded to the head 13. In this design, the head is produced with an opening to fit the electrode traces. The traces are attached to the head by an adhesive. There are multiple types of adhesives that could be used for this application. Some examples are: pressure sensitive adhesives (PSA), UV-curable adhesives, cyanoacrylates, urethane adhesives, hot-melt adhesives and epoxies.

Another embodiment for electrode trace attachment is a conductive ink electrode **14.** In this design, the electrode traces are applied on the head using conductive inks.

Another embodiment for electrode trace attachment is a flexible circuit **15** that is attached to the head thru a secondary operation. A flex circuit may be etched into a variety of shapes that can be bonded to the surface of the head. A flexible circuit **15** may be attached to some materials using a hot-melt adhesive such as a DuPont Pyralux. Flex circuits can be manufactured into numerous configurations that vary with regard to trace width, thickness, and spacing.

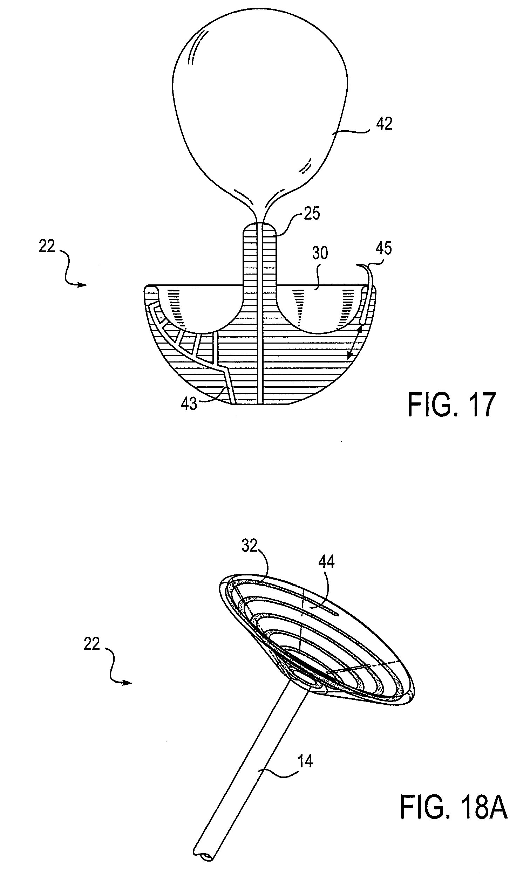

**Figure 17** provides a view of several embodiments of approaches by which to support apposition between an electrode-bearing surface **30** (electrodes are not shown in this figure) and cervical epithelial tissue **100** such that an appropriate level of therapeutic contact is made. Gaining an appropriate level of tissue apposition or therapeutically-appropriate contact is advantageous for an ablation procedure as the procedure may occur over the duration of several minutes, and stability of the tissue-electrode contact during this time is necessary. A clasping or bracketing feature, such as retractable tissue hooks **45,** for example, may be used to secure the device against the tissue while ablation is being performed. Alternatively, a balloon **42** may be deployed into the cervical os **102** or into the uterus to secure the ablation head against the tissue; inflation of the balloon **42** has the effect of pulling the ablation surface into close apposition with the cervical surface **100.** In yet another embodiment, a multi-channeled vacuum manifold **41** may be incorporated into the ablation head to draw the cervical tissue to the electrode on the ablation head surface **30.** The vacuum may be sourced from the generator or an external supply. In each of these alternative approaches to gaining tissue apposition methods described, it may be advantageous for the generator activate and deactivate the securement in conjunction with the start and finish of the ablative therapy.

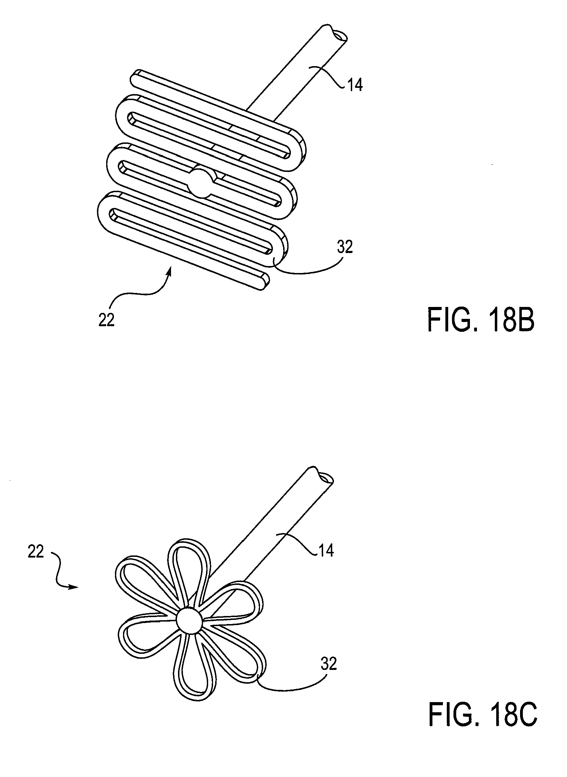

**Figures 18A - 18C** show alternative embodiments of an ablation probe head **22** that are particularly appropriate for single use, and as such are generally light in construction. **Figure 18A** shows a free-form electrode supported on a shaft **14** such that the bipolar traces **32** are separated by an insulative material **44.** The free-form electrode may be shaped in a variety of configurations in order to achieve a surface ablation on the cervix. Some potential shapes may be a spiral, which could be flat or concave. Another shape could be a sinusoidal pattern that follows a progressively larger spiral or rectangular path. Other shapes could also be achieved with this design. The insulative material could also be a material that holds the shape of the electrode, or it could have a shape-holding element attached or encased within it. The electrodes themselves may also be rigid enough to hold the shape of this design as shown in Figures **18B and 18C****..** Alternatively, the devices shown in **Figures 18A - 18C** could be monopolar and the electrode configuration constructed of a single or multiple electrodes.

Unless defined otherwise, all technical terms used herein have the same meanings as commonly understood by one of ordinary skill in the art of ablational technologies and treatment of neoplastic disease. While embodiments of the invention have been described in some detail and by way of exemplary illustrations, such illustration is for purposes of clarity of understanding only, and is not intended to be limiting. Moreover, any one or more features of any embodiment of the invention can be combined with any one or more other features of any other embodiment of the invention. For example, any type of electrode described or depicted in the context of one ablational energy element support surface configuration may be combined with any other ablational support surface configuration. Still further, it should be understood that the invention is not limited to the embodiments that have been set forth for purposes of exemplification.

## Claims

1. An ablation system (1) for treating abnormal epithelial tissue of a uterine cervix comprising:
an ablation device (10) comprising:
a handle (13);
a shaft (14) coupled with the handle at a proximal portion of the shaft, wherein the shaft is sized to be accommodated within a vagina and is of sufficient length to reach the uterine cervix from a natural opening of the vagina;
an operative head (22) coupled with the shaft at a distal portion of the shaft, the operative head having a radial orientation that is fixed relative to the handle, the operative head having a support surface (30) adapted to conformably engage at least a portion of the uterine cervix;
a plurality of radio frequency electrodes (32, 33) on the support surface that are configured to deliver ablational energy to the tissue in a manner that controls the surface area and depth of ablation, and
an energy generator (2) in electrical communication with the plurality of electrodes, the plurality of electrodes configured to receive energy from the energy generator,
**characterized in that** the plurality of electrodes are arranged in zones that are served by independently operable channels, and **in that** the system is configured to deliver radio frequency energy through the radio frequency electrodes to the uterine cervix at a power density that ranges between 5 W/cm² and 150 W/cm².

2. The system of claim 1, wherein the support surface (30) of the ablation device (10) is concave.

3. The system of claim 1, wherein the support surface (30) of the ablation device (10) comprises a center post (25) adapted to enter a cervical os.

4. The system of claim 1, wherein the plurality electrodes (32, 33) are circumferentially aligned on the support surface (30).

5. The system of claim 1, wherein the plurality of electrodes (32, 33) are configured into zones with different electrode densities.

6. The system of claim 1, wherein the plurality of electrodes (32, 33) are spaced apart at intervals in the range of about 0.1 mm to about 4 mm.

7. The system of claim 1, wherein the plurality of electrodes (32, 33) have a width in the range of about 0.1 mm to about 4 mm.

8. The system of claim 1, wherein the operative head (22) of the ablation device (10) comprises a rollable sheath (46) configured to unroll proximally to cover the shaft (14) of the ablation device.

9. The system of claim 1, wherein the distal portion of the shaft (14) of the ablation device (10) comprises a flexible portion configured to allow the support surface (30) of the operative head (22) to engage the uterine cervix.

10. The system of claim 1, wherein the distal portion of the shaft (14) of the ablation device (10) comprises an angled portion configured to allow the support surface (30) of the operative head (22) to engage the uterine cervix.

11. The system of claim 1, further comprising a feedback circuit that is operable to stop the delivery of the ablational energy from the energy generator (2) to the plurality of electrodes (32, 33) in response to an operational or sensed parameter.

12. The system of claim 1, wherein the plurality of electrodes are further configured to deliver radio frequency energy to the uterine cervix at an energy density that ranges between 5 J/cm². and 100 J/cm².

## Patentansprüche

1. Ablationssystem (1) zur Behandlung von einem abnormalen Epithelgewebe des Gebärmutterhalses, umfassend.
eine Ablationsvorrichtung (10), umfassend
einen Handgriff (13);
einen Schaft (14), der mit dem Handgriff an einem proximalen Abschnitt des Schaftes verbunden ist, wobei der Schaft dimensioniert ist, um in einer Scheide aufgenommen zu werden, und von ausreichender Länge, um den Gebärmutterhals von einer natürlichen Öffnung der Scheide aus zu erreichen; einen Handhabungskopf (22), der mit dem Schaft an einem distalen Abschnitt des Schafts verbunden ist, wobei der Handhabungskopf eine radiale Orientierung aufweist, die im Bezug zu dem Handgriff fixiert ist, wobei der Handhabungskopf eine Stützfläche (30) aufweist, die eingerichtet ist, mindestens in einen Abschnitt des Gebärmutterhalses anschmiegend einzugreifen;
mehrere Hochfrequenzelektroden (32, 33) an der Stützfläche, die konfiguriert sind, die Ablationsenergie in einer Weise an das Gewebe abzugeben, welche den Oberflächenbereich und die Tiefe der Ablation steuert;
und
einen Energiegenerator (2) in elektrischer Verbindung mit den mehreren Elektroden, wobei die mehreren Elektroden konfiguriert sind, Energie von dem Energiegenerator zu empfangen,
**dadurch gekennzeichnet, dass** die mehreren Elektroden in Zonen angeordnet sind, die durch voneinander unabhängig arbeitenden Kanälen versorgt werden, und dadurch, dass das System konfiguriert ist, eine Hochfrequenzenergie durch die Hochfrequenzelektroden an den Gebärmutterhals mit einer Leistungsdichte abzugeben, die im Bereich zwischen 5 W/cm² und 150 W/cm² liegt.

2. System nach Anspruch 1, wobei die Stützfläche (30) der Ablationsvorrichtung (10) konkav ist.

3. System nach Anspruch 1, wobei die Stützfläche (30) der Ablationsvorrichtung (10)
einen Mittelpfosten (25) aufweist, der eingerichtet ist, in einen Muttermund einzutreten.

4. System nach Anspruch 1, wobei die Mehrzahl der Elektroden (32, 33) umfangsseitig auf der Stützfläche (30) ausgerichtet sind.

5. System nach Anspruch 1, wobei die mehreren Elektroden (32, 33) in Zonen mit unterschiedlichen Elektrodendichten konfiguriert sind.

6. System nach Anspruch 1, wobei die mehreren Elektroden (32, 33) mit Zwischenräumen im Bereich von ungefähr 0,1 mm bis ungefähr 4 mm voneinander beabstandet sind.

7. System nach Anspruch 1, wobei die mehreren Elektroden (32, 33) eine Breite im Bereich von ungefähr 0,1 mm bis ungefähr 4 mm aufweisen.

8. System nach Anspruch 1, wobei der Handhabungskopf (22) der Ablationsvorrichtung (10) eine abrollbare Hülse (46) aufweist, die zum proximalen Ausrollen für das Ummanteln des Schafts (14) der Ablationsvorrichtung konfiguriert ist.

9. System nach Anspruch 1, wobei der distale Abschnitt des Schaftes (14) der Ablationsvorrichtung (10) einen flexiblen Abschnitt aufweist, der konfiguriert ist, es der Stützfläche (30) des Handhabungskopfes (22) zu ermöglichen, mit dem Gebärmutterhals in Eingriff zu treten.

10. System nach Anspruch 1, wobei der distale Abschnitt des Schaftes (14) der Ablationsvorrichtung (10) einen abgewinkelten Abschnitt aufweist, der konfiguriert ist, es der Stützfläche (30) des Handhabungskopfes (22) zu ermöglichen, mit dem Gebärmutterhals in Eingriff zu treten.

11. System nach Anspruch 1, ferner umfassend eine Rückkopplungsschaltung, die betreibbar ist, die Zuführung der Ablationsenergie von dem Energiegenerator (2) zu den mehreren Elektroden (32, 33) in Reaktion auf einen Betriebsparameter oder einen erfassten Parameter zu stoppen.

12. System nach Anspruch 1, wobei die mehreren Elektroden ferner konfiguriert sind, eine Hochfrequenzenergie an den Gebärmutterhals mit einer Energiedichte abzugeben, die im Bereich zwischen 5 J/cm² und 100 J/cm² liegt.

## Revendications

1. Système d'ablation (1) pour le traitement d'un tissu épithélial anormal du col de l'utérus comprenant :
un dispositif d'ablation (10) comprenant :
une poignée (13) ;
une tige (14) couplée à la poignée dans une partie proximale de la tige, dans lequel la tige est calibrée pour être reçue dans un vagin et a une longueur suffisante pour atteindre le col de l'utérus par une ouverture naturelle du vagin ;
une tête opératoire (22) couplée à la tige dans une partie distale de la tige, la tête opératoire ayant une orientation radiale qui est fixe par rapport à la poignée, la tête opératoire ayant une surface de support (30) adaptée pour s'engager en conformité sur au moins une partie du col de l'utérus ;
une pluralité d'électrodes de fréquence radio (32, 33) sur la surface de support qui sont configurées pour délivrer de l'énergie d'ablation au tissu de manière à régler la surface spécifique et la profondeur d'ablation, et
un générateur d'énergie (2) en communication électrique avec la pluralité d'électrodes, la pluralité d'électrodes étant configurées pour recevoir de l'énergie du générateur d'énergie, **caractérisé en ce que** la pluralité d'électrodes sont aménagées dans des zones qui sont desservies par des canaux indépendamment opérationnels et **en ce que** le système est configuré pour délivrer de l'énergie de fréquence radio à travers les électrodes de fréquence radio au col de l'utérus à une densité de puissance qui se situe dans une plage de 5 W/cm² à 150 W/cm².

2. Système selon la revendication 1, dans lequel la surface de support (30) du dispositif d'ablation (10) est concave.

3. Système selon la revendication 1, dans lequel la surface de support (30) du dispositif d'ablation (10) comprend un montant central (25) adapté pour pénétrer dans un orifice cervical.

4. Système selon la revendication 1, dans lequel la pluralité d'électrodes (32, 33) sont alignées circonférentiellement sur la surface de support (30).

5. Système selon la revendication 1, dans lequel la pluralité d'électrodes (32, 33) sont configurée en zones ayant différentes densités d'électrodes.

6. Système selon la revendication 1, dans lequel la pluralité d'électrodes (32, 33) sont espacées l'une de l'autre à intervalles dans la plage d'environ 0,1 mm à environ 4 mm.

7. Système selon la revendication 1, dans lequel la pluralité d'électrodes (32, 33) ont une largeur dans la plage d'environ 0,1 mm à environ 4 mm.

8. Système selon la revendication 1, dans lequel la tête opératoire (22) du dispositif d'ablation (10) comprend une gaine enroulable (46) configurée pour se dérouler de manière proximale afin de couvrir la tige (14) du dispositif d'ablation.

9. Système selon la revendication 1, dans lequel la partie distale de la tige (14) du dispositif d'ablation (10) comprend une partie flexible configurée pour permettre à la surface de support (30) de la tête opératoire (22) de s'engager dans le col de l'utérus.

10. Système selon la revendication 1, dans lequel la partie distale de la tige (14) du dispositif d'ablation (10) comprend une partie inclinée configurée pour permettre à la surface de support (30) de la tête opératoire (22) de s'engager dans le col de l'utérus.

11. Système selon la revendication 1, comprenant en outre un circuit de rétroaction qui peut opérer pour arrêter la fourniture de l'énergie d'ablation du générateur d'énergie (2) à la pluralité d'électrodes (32, 33) en réponse à un paramètre opérationnel ou détecté.

12. Système selon la revendication 1, dans lequel la pluralité d'électrodes sont en outre configurées pour délivrer de l'énergie de fréquence radio au col de l'utérus à une densité d'énergie qui se situe dans la plage de 5 J/cm² à 100 J/cm².
